Europäisches Patentamt

European Patent Office

Office européen des brevets

(11) Veröffentlichungsnummer: **0 200 673**

A2

(12) # EUROPÄISCHE PATENTANMELDUNG

(21) Anmeldenummer: **86730070.9**

(22) Anmeldetag: **29.04.86**

(51) Int. Cl.⁴: **G 01 N 27/28**

(30) Priorität: **02.05.85 DE 3516018**

(43) Veröffentlichungstag der Anmeldung:
**05.11.86 Patentblatt 86/45**

(84) Benannte Vertragsstaaten:
**AT BE CH DE FR GB IT LI LU NL SE**

(71) Anmelder: **MIC AG**
**Freigutstrasse 24**
**CH-8002 Zürich(CH)**

(72) Erfinder: **Kruse, Holger**
**Kurfürstendamm 64/65**
**D-1000 Berlin 15(DE)**

(72) Erfinder: **Merki, Hans, Dr.-med.**
**Ansbacherstrasse 55**
**D-1000 Berlin 30(DE)**

(74) Vertreter: **Pfenning, Meinig & Partner**
**Kurfürstendamm 170**
**D-1000 Berlin 15(DE)**

(54) **Tragbares Gerät zur Feststellung der Magenfunktion auf der Grundlage der Magensäure und Verfahren zu seiner Anwendung.**

(57) Es wird ein tragbares Gerät zur Feststellung der Magenfunktion auf der Grundlage der Magensäure vorgeschlagen. Das Gerät weist eine pH-Sonde zur kontinuierlichen Messung von pH-Werten im Oesophagus und im Magen auf, weiterhin ist ein Meßwertspeicher zur Speicherung der einzelnen Meßwerte in Zuordnung einer von einer elektronischen Uhr gelieferten Uhrzeit vorgesehen. Eine Anzeigeeinheit zeigt die Meßwerte kontinuierlich an und ein Drucker gibt die Meßwerte während der Messung in Abhängigkeit von der Zeit aus. Bestimmte Schwellenwerte, die über ein Tastenfeld eingebbar sind, werden in einem Schwellenwertspeicher gespeichert. Ein Vergleicher vergleicht die jeweiligen Schwellenwerte mit den momentanen Meßwerten oder mit einem aus den Meßwerten über eine bestimmte Zeit gebildeten Mittelwert. Bei Über- bzw. Unterschreiten des Schwellenwertes wird ein Signal abgegeben, das einen Signalgeber aktiviert.

Croydon Printing Company Ltd.

<u>Tragbares Gerät zur Feststellung der Magenfunktion
auf der Grundlage der Magensäure und Verfahren
zu seiner Anwendung</u>

Die Erfindung betrifft ein tragbares Gerät zur Feststellung der Magenfunktion auf der Grundlage der Magensäure, wobei der Säurespiegel über den pH-Wert der Magenflüssigkeit gemessen wird.

Um den Säuregehalt der Magenflüssigkeit festzustellen, ist es bekannt, in mehreren Abständen über eine Sonde mit Schlauch die Magenflüssigkeit zu entnehmen, die anschließend im Labor auf die $H^+$-Ionenaktivität bzw. auf den pH-Wert untersucht wird. Dieses Verfahren ist äußerst aufwendig, wobei bei der Entnahme der Proben Verunreinigungen in diese gelangen können, die den eigentlichen Säurespiegel verfälschen. Da sich die Säuresekretion zeitlich stark ändert, müßten zur genauen Erfassung des Säuresekretionsverhaltens laufend Proben entnommen werden, eine Vorgehensweise, die den Probanden in starkem Maße in seinem Wohlbefinden beeinträchtigt und keinen physiologischen Tagesablauf mit Ernährung des Patienten zuläßt. Der Erfindung liegt die Aufgabe zugrunde, ein kleines tragbares Gerät zur Feststellung der Magenfunktion auf der Grundlage der Magensäure zu schaffen, das kostengünstig in seiner Herstellung ist, ohne zusätzliche Kenntnisse anwendbar und überall einsetzbar ist, wobei gleichzeitig zu einer kontinuierlichen Messung eine Auswertung der Messwerte vorgenommen wird.

Diese Aufgabe wird erfindungsgemäß durch die Merkmale des Hauptanspruchs gelöst. Durch Vorsehen des erfindungsgemäßen tragbaren Gerätes, bei dem die von einer Sonde kontinuierlich über einen längeren Zeitbereich gelieferten pH Messwerte gespeichert, ausgewertet, angezeigt und ausgedruckt werden, stehen zu jedem Zeitpunkt der Messung und auch unmittelbar danach die gesamte Information zur Verfügung, so daß, falls notwendig, sofort Maßnahmen zur Behandlung getroffen werden können. Die kleine Ausführung des Gerätes beeinträchtigt den Probanden trotz langer Messzeit nur unwesentlich in seinen Aktivitäten. Das Gerät erlaubt, die individuelle Tagesrhytmik im Säuresekretionsverhalten zu beobachten, wobei die Auswirkung verschiedener Einflußgrößen auf den Säurespiegel, wie eingenommene Nahrungsmittel, Zigaretten, Alkohol, Kaffee oder Medikamente sofort erkannt werden kann. Diese Kenntnis erlaubt eine Abstimmung der Medikamenteneinnahme auf die Essensgewohnheiten sowie die Tagesaktivitäten des Probanden, so daß der Medikamentenverbrauch verringert bzw. unnötige Medikamente nicht eingenommen werden müssen. Außerdem erlaubt das erfindungsgemäße Gerät den Einsatz verschiedener Medikamente innerhalb einer einzigen Messung, wodurch Medikamenten-Responder von Non-respondern zuverlässig unterschieden werden können und in einer geringstmöglichen Messzeit das für den jeweiligen Probanden für seinen Säurespiegel günstige Medikament herausgefunden werden kann.

Dadurch, daß in einem Speicher über das Tastenfeld eingebbare Schwellenwerte gespeichert werden und ein Vergleicher vorgesehen ist, der die jeweiligen Schwellenwerte mit den Messwerten vergleicht, wobei bei momentanem oder länger-währendem Unter- bzw. Überschreiten des jeweiligen Schwellenwertes ein akustisches Signal abgegeben wird, kann eine sofortige Maßnahme zur Abänderung des nicht normalen Säureverhaltens vorgenommen werden.

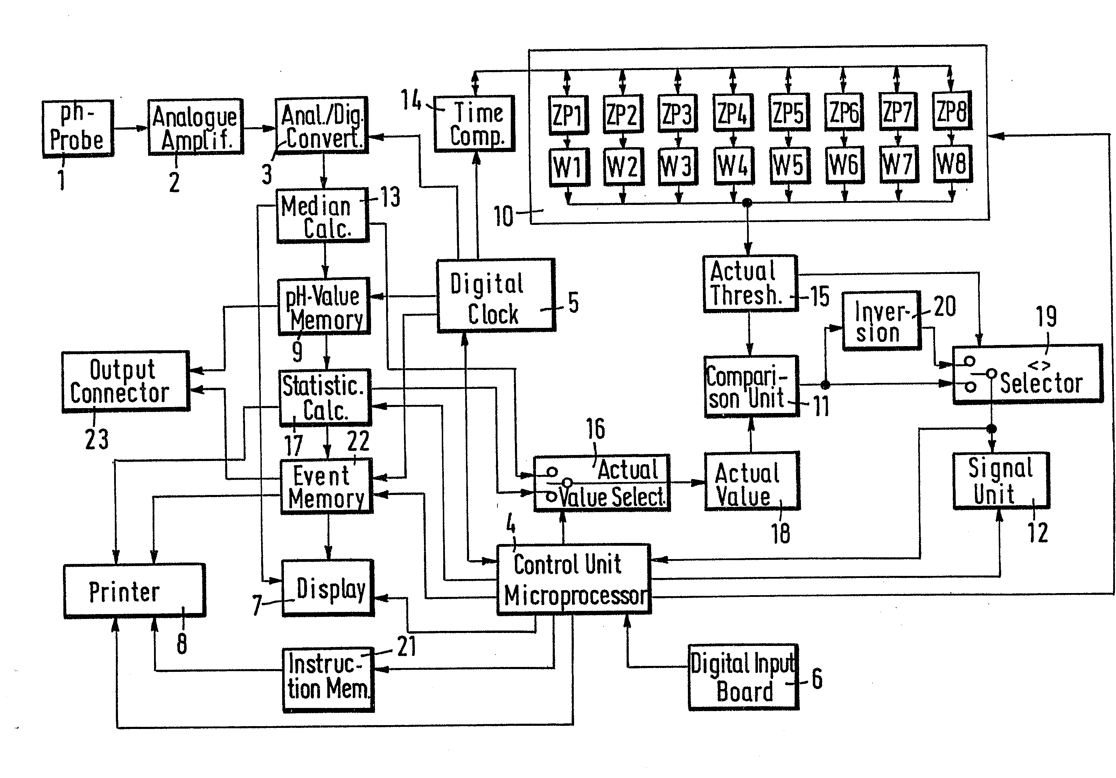

Ein weiterer Vorteil des erfindungsgemäßen Gerätes liegt darin, daß der Proband bei Auftreten einer Symptomatik, z.B. von Schmerzen über die sofort ausgedruckte Information über den Säurespiegel erkennt, ob seine eigenen Beschwerden an einem unnatürlichen pH- liegen. Dadurch kann die Einnahme von Medikamenten wegen Schmerzen, die nicht durch den Säurespiegel hervorgerufen werden, verhindert werden. Die Erfindung ist in der Zeichnung dargestellt und wird in der nachfolgenden Beschreibung näher erläutert. Die einzige Figur zeigt ein Blockschaltbild des erfindungsgemäßen Gerätes.

Die Sonde 1, die eine pH-sensitive Elektrodenanordnung aufweist, ist mit einem Analog-Verstärker 2 verbunden, der an einen A/D-Wandler 3 angeschlossen ist. Der A/D-Wandler wird von einer Steuereinheit 4 gesteuert, an die eine Uhr 5 mit Kalender angeschlossen ist, die die zeitliche Steuerung vornimmt. Weiterhin ist ein Tastenfeld 6, das als Folientastenfeld ausgebildet sein kann, eine Anzeigeeinheit 7 und ein graphikfähiger Nadeldrucker 8 vorgesehen, die alle mit der Steuereinheit 4 in Verbindung stehen. Ein programmierbarer Messwertspeicher 9 und ein programmierbarer Schwellenwertspeicher 10 sind an die Steuereinheit angeschlossen,und ein Vergleicher 11 erhält an seinem einen Eingang die Messwerte und an seinem anderen Eingang die Schwellenwerte. Der Vergleicher 11 steht mit einem Signalgeber 12 in Verbindung, der als akustischer Signalgeber mit Piezoscheibe ausgebildet sein kann.

Das von der Sonde 1 gelieferte kontinuierliche Signal wird von dem Analog-Verstärker 2 verstärkt und aufgearbeitet und steht als digitales Signal am Ausgang des A/D-Wandlers 3 zur Verfügung. Abhängig von einer beispielsweise über das Tastenfeld 6eingestellten Abtastrate tastet die Steuereinheit 4 unter Steuerung durch die Uhr 5 den Ausgang des A/D-Wandlers 3 ab und leitet die Messwerte an einen Medianwertbildner 13 weiter. Innerhalb der eingestellten Abtastrate liefert der A/D-Wandler 3 ungefähr 100 Messwerte. Der Medianwertbildner 13 sortiert diese Messwerte nach ihrer Größe und wählt dann den mittleren Messwert, beispielsweise den 51igsten aus. Dieser Messwert steht am Ausgang des Medianwertbildners 13 als Medianwert an und wird dem Messwertspeicher 9 zugeführt, in dem er zeitrichtig gespeichert wird.

Vorzugsweise vor der kontinuierlichen Messung werden über das Tastenfeld 6 und die Steuereinheit 4 Schwellenwerte bzw. Grenzwerte für die pH-Werte der Magenflüssigkeit des Probanden eingegeben, die in dem Schwellenwertspeicher 10 gespeichert werden. Im Blockschaltbild sind beispielhaft acht Schwellenwerte, Sch W1 bis Sch W8, gezeigt. Jedem Schwellenwert ist ein Zeitpunkt ZP 1 bis ZP 8 zugeordnet. Außerdem enthalten die Schwellenwerte die Information, ob der Signalgeber 12 bei Über- oder Unterschreiten des Schwellenwertes aktiv werden soll. Mit dieser Information wird eine Kleiner-Größer Auswahleinheit 19 gesteuert.

Ein Zeitvergleicher 14 vergleicht die in den Schwellenwertspeicher 10 eingegebenen Zeitpunkte mit der durch die Uhr 5 vorgegebenen Uhrzeit und wählt abhängig von seinem Vergleichsergebnis den aktuellen Schwellenwert aus, der in einem Zwischenspeicher 15 gespeichert wird. Selbstverständlich können in dem Schwellenwertspeicher 10 auch zeitunabhängige Schwellenwerte gespeichert werden, die abhängig von dem Steuersignal der Steuereinheit 4 jeweils dem Zwischenspeicher 5 zugeführt werden.

Der Messwertspeicher 9 ist mit einem statistischen Rechner 17 verbunden, der beispielsweise die Häufigkeitsverteilung der gemessenen pH-Werte, den Prozentsatz der gemessenen pH-Werte, die über einem bestimmten Bezugs- oder Schwellenwert liegen und/oder den Mittelwert über einen vorgebbaren Zeitraum oder dergleichen berechnet.

Der Ausgang des statistischen Rechners 17 und der Ausgang des Medianwertbildners 13 sind mit einer beispielsweise als Schalter ausgebildeten Ist-Wert Auswahleinheit 16 verbunden, die abhängig von einem von der Steuereinheit 4 gelieferten Steuersignal zwischen den Ausgängen des statistischen Rechners 17 und des Medianwertbildners 13 hin- und herschaltet und das jeweilige Ausgangssignal an einen Ist-Wert-Speicher 18 weiterleitet. An dem Vergleicher 11 liegt somit der aktuelle pH-Messwert oder ein Mittelwert über einen festen Zeitraum, die jeweils mit dem entsprechenden Schwellenwert verglichen werden. Auf diese Weise kann neben momentanen Änderungen auch festgestellt werden, ob innerhalb einer vorgegebenen Zeit, beispielsweise 90 Minuten, die Acidität der Magenflüssigkeit stark erhöht ist. Unterschreitet bzw. überschreitet der Ist-Wert den Schwellenwert, so ändert sich das Ausgangssignal des Vergleichers 11. Der Ausgang des Vergleichers 11 ist einmal direkt und zum anderen über einen das Signal des Vergleichers 11 umkehrenden Inverter 20 mit der Kleiner-Größer-Auswahleinheit 19 verbunden, die als Schalter ausgebildet sein kann und abhängig von dem im Zwischenspeicher 15 gespeicherten Signal, das neben dem Schwellenwert auch die Information über das etwaige Über- oder Unterschreiten des Schwellenwertes enthält, zwischen den Ausgängen des Vergleichers 11 und des Inverters 20 umschaltet und das jeweilige Signal an die Steuereinheit 4 und den Signalgeber 12 weiterleitet, der gegebenenfalls einen Warnton abgibt, wobei die Steuereinheit 4 entsprechend die Anzeigeeinheit 7 und den Drucker 8 ansteuert.

Mit der Steuereinheit 4 ist ein Anweisungsspeicher 21 verbunden, der bestimmte Anweisungen für den Probanden gespeichert hat, beispielsweise "trinke Milch" oder "nimm Medikament ein", wobei die jeweiligen Anweisungen von der Steuereinheit 4 beispielsweise abhängig von der Uhrzeit oder von dem Signal des Signalgebers 12 ausgelesen werden und von dem Drucker 8 oder der Anzeigeeinheit 7 sichtbar gemacht werden.

Weiterhin ist ein Ereignisspeicher 22 vorgesehen, in dem während der Messung eintretende Ereignisse zeitrichtig gespeichert werden. So wird das Aktivieren des Signalgebers 12 durch den Vergleicher 11 festgehalten. Das Tastenfeld 6 enthält neben den Ziffern 0 bis 9 auch Symbole, beispielsweise das einer Zigarette oder einer Tasse oder eines Medikaments.

Mit Hilfe dieser Symbole kann das Befolgen einer angezeigten Anweisung durch Drücken der jeweiligen Taste quittiert werden. Sowohl die Quittierung als auch bestimmte die Messung beeinflussende Ereignisse, wie das Einnehmen einer Mahlzeit, das Rauchen einer Zigarette oder dergleichen können in dem Ereignisspeicher 22 festgehalten werden.

Die gemessenen pH-Werte werden kontinuierlich auf der Anzeigeeinheit 7 dargestellt und/oder von dem Drucker 8 ausgedruckt, der sie unter anderem in Abhängigkeit von der Zeit aufzeichnet. In einer derartigen Verlaufskurve werden alle im Ereignisspeicher 22 festgehaltenen Daten zeitrichtig registriert, so daß ein vollständiges Messprotokoll vorhanden ist. Entsprechend werden alle vom statistischen Rechner 17 berechneten Daten ausgedruckt.

Falls die pH-Sonde 1 während der Messung verrutscht, ändern sich die pH-Messwerte und verfälschen die Messung. Durch Eingeben und Abrufen bestimmter Schwellenwerte kann über den Vergleicher 11 und die Kleiner-Größer Auswahleinheit 19 diese Änderung festgestellt und durch einen Warnton kenntlich und eine entsprechende Anzeige sichtbar gemacht werden. Somit können ungültige Messungen sofort erkannt und verhindert werden.

Das Gerät weist eine Schnittstelle 23 auf, über die die Daten des Messwertspeichers 9 und des Ereignisspeichers 22 einer Datenverarbeitungsanlage nach Beendigung der Messung zugeführt und dort weiter verarbeitet werden können.

In einem anderen Ausführungsbeispiel ist eine als Infusionspumpe ausgebildete Medikamentenpumpe vorgesehen, die abhängig von dem gemessenen pH-Wert bzw. dem Signal des Vergleichers 11 betätigt wird, wobei nach erfolgter Infusion ein Quittierungssignal im Ereignisspeicher 22 gespeichert wird.

Im folgenden werden Beispiele genannt, wie das erfindungsgemäße Gerät in der Diagnose und der Therapie bezüglich der Säurekonzentration verwendet werden kann.

1. Diagnose eines oberen gastrointestinalen, erosiven und/oder entzündlichen Ulcusleiden die sich auf die Hypersekretion von HCl beziehen.

Beispiel 1:

In einer klinischen Studie von 24 Patienten mit unbehandeltem Magengeschwür wurde gefunden, daß das aktuelle Ulcusleiden, endoskopisch nachweisbar, einherging mit:

a) einem mittleren pH-Wert von weniger als 1,5 über einen Meßzeitraum von 24 Stunden;

b) einem Befund, daß mehr als 60 % aller über 24 Stunden gemessener pH-Werte weniger als 1,5 betrugen;

c) einer Dauer des intragastrischen pH-Wertes weniger als 1,5 von mehr als 12 Stunden innerhalb des 24 Stunden Zeitraumes;

d) einem intragastrischen pH-Wert von weniger als 1,5, insbesondere innerhalb der Stunden von $19^{00}$ Uhr bis $3^{00}$ Uhr.

Zusätzlich steigert die Nahrungsaufnahme durch normale Patienten immer den intragastrischen pH-Wert von einem Bereich von 1,5 bis 3,0 vor der Mahlzeit auf einen pH-Wert nach der Mahlzeit von 4,0 oder höher. Patienten mit Ulcusleiden zeigen einen geringeren Anstieg des pH-Wertes nach der Mahlzeit als andere Patienten. Dies kann sich auf ein größeres Volumen der Säuresekretion beziehen. Während die oben angeführten Kriterien auch durch häufiges Entnehmen der Luminalflüssigkeit und externes Messen der pH-Werte oder unter Verwendung reiner Speicher und Verarbeiten der Daten mit einem externen Computer bestimmbar sind, kann mit der vorliegenden Erfindung die Diagnose sehr viel schneller und durch jeden Arzt getroffen werden und Daten können unter physiologischen und ambulanten Bedingungen gesammelt werden.

2. Bestimmung des Tagesrhythmus der HCl Sekretion.

Beispiel 2:

Jeder Mensch scheint seinen spezifischen Tagesrhythmus für die Magensäure zu haben. Es läßt sich schließen, daß zwei Gruppen bestehen:

a) solche mit gleichmäßig hoher Magensäure während der Nacht;und

b) solche mit Änderungen des pH-Wertes während des gleichen Zeitraums.

Es existieren nur ungenügende Daten für "normale" Personen ohne Ulcusleiden, so daß nicht mit Gewißheit das Bestehen dieser zwei Untergruppen festgelegt werden kann oder gesagt werden kann, wie konstant ein gemessener Rhythmus über die Zeit ist. Andererseits wurde eine enge Beziehung zwischen einem niedrigen pH-Wert kleiner als 1,5 über einen längeren Zeitraum, insbesondere wenn dieser niedrige pH-Wert während der Nacht auftritt und der Entwicklung von floridem Ulcus oder Erosionen beobachtet. Das erfindungsgemäße Gerät kann leicht und schnell den Tagesrhythmus eines Patienten dokumentieren, wodurch dem Arzt bei der Bestimmung und Durchführung der Therapie geholfen wird. Beispielsweise kann der Tagesrhythmus durch Messungen des pH-Wertes über 24 Stunden oder länger, durch Berechnung der mittleren pH-Werte während vorbestimmter Zeiträume und durch Vergleichen der mittleren pH-Werte mit Daten von normalen Patienten bestimmt werden.

3. Behandlung einer Ulcuserkrankung; Zeitpunkt.

Beispiel 3:

Es wurde von Ireland R., Colin-Jones D.G. et al., Lancet ii, 274, 1984 berichtet, daß eine einzige amtliche Dosis eines H-2-Rezeptorblockers, eingenommen um $22^{00}$ Uhr, ebenso wirksam für die Heilung eines floriden Ulcus ist wie die gleiche Gesamtdosis zweimal täglich. Diese im allgemeinen anerkannte Praxis geht von einem allgemeinen täglichen Säurerhythmus in Patienten mit Ulcuserkrankungen und einer Abdeckung der starken nächtlichen Säure im Magen durch die obige Dosierung aus. In einem doppeltverdeckten, zufallsverteilten, überwachten klinischen Versuch mit 12 gesunden Freiwilligen und 24 Patienten mit endoskopisch nachgewiesenen, vor dem Versuch nicht therapierten Ulcuserkrankungen wurde der tägliche Rhythmus der Magensäure gemessen. Die Wirkung auf diese Rhythmen einer abendlichen Dosis H-2-Rezeptorblocker (vs. Placebo), eingenommen entweder um 18$^{00}$ Uhr oder um 22$^{00}$ Uhr, wurde in einer weiteren, ähnlich angelegten Studie untersucht. Es wurde gefunden, daß die Vorlaufzeit vor der Hemmung der Säureproduktion durch den H-2-Rezeptorblocker länger ist als von Ireland et al angenommen, das heißt, ein um 22$^{00}$ Uhr verabreichtes Medikament wirkte erst nach Mitternacht, bis zu diesem Zeitpunkt wurde ein hoher Säuregehalt im Magen festgestellt; ein um 18$^{00}$ Uhr verabreichtes Medikament war wirksamer, da es den gesamten Zeitraum und der Nacht bis zum Morgen abdeckte. Dies zeigt, wie wichtig die Kenntnis des Tagesrhythmus jedes Patienten und die Wirkung einer Medikamententherapie auf diesen Rhythmus zur Bestimmung einer optimalen Therapie ist. Eine optimale Therapie bedeutet eine Verabreichung nur einer solchen Menge von Medikamenten, wie sie notwendig ist, um die Zeiträume der Gefährdung der Magenschleimhäute abzudecken.

4. Welche Therapie soll angewendet werden.

Beispiel 4:

Im allgemeinen kann neben der Operation (Vagotomie, Resektion) eine Anzahl von Maßnahmen in der Behandlung von Ulcuserkrankungen getroffen werden. Diese Maßnahmen sind entsprechend der Reihenfolge der Schwere der Erkrankung:

Diät und Änderung und Beeinflussung der Lebensführung, Antazide und H-2-Rezeptorblocker.
Diese Liste betrifft nicht den Blutungszustand,
der andere Maßnahmen verlangt oder die Tatsache,
daß einige andere Medikamente wirksam mit
H-2-Rezeptorblockern kombiniert werden können,
um die Wirksamkeit der Behandlung dieses Zustandes
zu erhöhen. Es sind allerdings viele Antazidum-
Präparate auf dem Markt und weniger, aber immer
noch viel H-2-Rezeptorblocker-Medikamente erhältlich.
Die Verwendung der beschriebenen Erfindung kann
den Arzt mit detaillierten und schnellen
Informationen versehen über:

a)  ob ein Antazidum verwendet werden soll und
    wenn ja, welches Antazidum dem Patienten
    wirksam eine "Normalisierung" des pH-Wertes
    der Magensäure und nicht nur symptomatische
    Erleichterung bringt, und


b)  ob ein H-2-Rezeptorblocker verwendet werden
    soll und wenn ja, welcher Blocker zur geeigneten
    Zeit verwendet wird. Patienten mit endoskopisch
    bestätigten Ulcuserkrankungen wurden vor
    der Medikamentenbehandlung dahingehend
    beobachtet, daß sie niedrige pH-Werte der
    Magensäure während der Tagesstunden ($6^{00}$ Uhr
    bis $18^{00}$ Uhr) mit nur geringer Pufferwirkung
    (pH-Wertsteigerung) während der Mahlzeiten,
    aber schwankende nächtliche pH-Werte mit
    Mittelwerten größer als 1,5 aufwiesen.
    Derartige Patienten sprechen gut auf Antazida
    an und benötigen keine H-2-Rezeptorblocker.
    Patienten mit endoskopisch bestätigten
    Ulcuserkrankungen, die einen verlängerten
    pH-Mittelwert von weniger als 1,5 während der
    Nacht ($18^{00}$ Uhr bis $6^{00}$ Uhr) aber unterschiedliche

pH-Werte mit signifikanter Pufferwirkung während der Tageszeiten aufweisen,
· reagieren am besten auf H-2-Rezeptorblocker. Die vorliegende Erfindung kann daher dazu verwendet werden, um das pH-Wertprofil eines Patienten zur Feststellung einer wirksamen Medikamentenbehandlung zu bestimmen.

5. Prophylaktische Verwendung der Erfindung.

Während Antazida für die Patienten mit ulcus-ähnlichen Symptomen ohne Verschreibung frei erhältlich sind, sind H-2-Rezeptorblocker und ähnliche Medikamente nur auf Verschreibung erhältlich und nur dann, wenn eine definierte, endoskopisch oder röntgenologisch bestätigte Diagnose eines Magenulcus vorhanden ist. Da ein Magenulcus wegen Blutungen oder möglicher Perforation lebensbedrohend werden kann, erhebt sich die Frage, ob die prophylaktische Anwendung ein Teil der optimalen Therapie sein sollte. Dies ist insbesondere für Patienten wichtig, bei denen der erste nachgewiesene Ulcus abgeheilt ist.

Beispiel 5:

In einem überwachten klinischen Versuch wurden 35 Patienten mit einem ersten abgeheilten Ulcus durch häufige Endoskopie und einer 24 Stunden pH-Wert-Überwachung während dreier Monate nachuntersucht, wobei die gleichen pH-Wertkriterien der Diagnose für Ulcuserkrankung wie im Beispiel 1 verwendet und mit dem Auftreten neuer Ulcuserkrankungen oder Erosionen verglichen wurden. In der Gruppe betrug das Wiederauftreten im vorgegebenen Zeitraum 23 bis 29 % und es gab eine gute Korrelation zwischen den pH-Wert-Kriterien aus Beispiel 1. Selbstverständlich muß eine sehr viel größere Population derartiger Patienten untersucht werden, um genauer bestimmen zu können, wieviel geheilte Patienten mit einem ersten Ulcus chronische Ulcuspatienten werden. Die Verwendung der vorliegenden Erfindung wird somit wichtig für die Auswahl derjenigen Patienten, die eine prophylaktische Behandlung mit H-2-Rezeptorblocker zur Verhinderung von Rückfällen verlangen.

6.  Die Wirkung der Mahlzeiten und einer Diät
    bei Ulcuserkrankungen.

Jede Mahlzeit weist eine Pufferwirkung auf den Magen und eine Volumenwirkung sowohl von der Mahlzeit selbst als auch von dem erzeugten Sekretionsvolumen auf. Unabhängig von der Zusammensetzung der Mahlzeit liegen die pH-Werte der Magensekretionen vor der Mahlzeit im Bereich von 2,0 bei normalen Patienten und bei 0,9 bis 1,5 bei Patienten mit Ulcuserkrankungen. Die Mahlzeit bewirkt höhere pH-Werte für eine oder mehrere Stunden, bis 4,0 oder höher. Es besteht eine Tendenz bei Patienten mit Ulcuserkrankungen, einen geringeren pH-Wertanstieg bei einer Standardmahlzeit zu zeigen als bei Gesunden.

Beispiel 6:

Es wurden bei 24 Patienten mit endoskopische nachgewiesenem Ulcus (nicht unter Medikamentenbehandlung) und 12 Gesunden über 24 Stunden die pH-Kurven aufgenommen, wobei alle die gleichen Mahlzeiten einnahmen. Jede Gruppe wies zu gleichen Teilen Männer und Frauen auf. Folgender Vergleich der mittleren pH-Werte als Wirkung auf die Mahlzeiten ergab sich:

|  | zu Ulcuserkrankungen neigende Patienten | Gesunde |
|---|---|---|
| pH-Wert vor der Mahlzeit, $16^{00}$ Uhr | 1,5 | 1,9 |
| pH-Wert nach der Mahlzeit, $18^{00}$ Uhr | 1,6 | 3,9 |
| Dauer der Wirkung der Mahlzeit in Stunden | 1 | 4 |
| pH-Wert vor der Mahlzeit, $19^{00}$ Uhr | 1,5 | 1,5 |
| pH-Wert nach dem Frühstück, $7^{00}$ Uhr | 2.0 | 3,5 |
| Dauer der Wirkung des Frühstücks in Stunden | 2 | 4 |

Die Verwendung der vorliegenden Erfindung ermöglicht dem Arzt, die Wirkung der Mahlzeiten auf den pH-Wert in Beziehung auf den gesamten täglichen Rhythmus zu messen, den Patienten als therapeutische Maßnahme anzuweisen, wann die Mahlzeiten eingenommen werden sollen, zu testen, ob verschiedene Diäten den Grad und die Dauer der Pufferwirkung verbessern und den Patienten eine Diät zu verschreiben.

7. Die Wirkung von Kaffee auf die Magensäure.

Beispiel 7:

In einer zufallsverteilten, doppeltverdeckten überwachten Studie von 10 gesunden Freiwilligen wurden folgende Lösungen während der pH-Wert-überwachung in konstanten Volumendosen verabreicht: Kaffee, koffeinfreier Kaffee vom gleichen Hersteller und heißes Wasser als Kontrolle. pH-Wertmessungen wurden vor und 6 Stunden nach dem Trinken durchgeführt. Für eine kurze Zeitdauer stieg aufgrund der Volumenwirkung der pH-Wert, gefolgt durch die bekannte Säureerhöhung. Die Post-Volumen-Artefakt-pH-Werte wurden verglichen mit folgendem Ergebnis:

a) koffeinhaltiger Kaffee erzeugt eine signifikant größere Azidität als Wasser oder koffeinfreier Kaffee, und

b) der Unterschied zwischen Wasser und koffeinfreiem Kaffee war nicht signifikant.

Es gibt individuelle Unterschiede in der Wirkung von Koffein auf die pH-Werte. Die Verwendung der vorliegenden Erfindung ermöglicht dem Arzt zu bestimmen, ob ein bestimmter Patient normalen Kaffee trinken kann und, falls erlaubt, zu welchem Zeitpunkt bei Berücksichtigung des täglichen pH-Wert-Rhythmus des Patienten.

8. Die Wirkung des Rauchens bei Ulcuserkrankungen.

Dasdurch das Rauchen aufgenommene Nikotin ist nicht immer ein starkes HCl sekretionsanregendes Mittel. Allerdings tauchen individuelle Unterschiede auf. Die Möglichkeit des Markierens von Ereignissen bei der vorliegenden Erfindung kann den Arzt darüber informieren, ob die Rauchgewohnheiten des Patienten die Ulcuserkrankung verschlimmert oder nicht.

9. Der Effekt des Streß.

Streß am Arbeitsplatz oder zu Hause ist bekannt als auslösender Faktor einer Ulcus- oder Erosionsbildung. Obwohl bisher keine Studien unter Verwendung der 24 stündigen pH-Wertüberwachung durchgeführt wurden, um dieses Phänomen zu beobachten, so diktiert doch die Logik, daß der Vergleich der 24 Stunden Kurven und pH-Wertdaten desselben Patienten in und außerhalb der Streßumgebung dem Arzt ein Mittel zur Feststellung des Effektes auf den Patienten zur Verfügung stellt, so daß dieser ihn auf mögliche Änderungen der Lebensbedingungen aufmerksam machen kann.

10. Die Wirksamkeit von Operationen auf Ulcuserkrankungen.

Im allgemeinen werden zwei Arten von Operationen bei verschiedenen Stufen der Ulcuserkrankung durchgeführt: die Vagotomie für Patienten, die müde von der kontinuierlichen Medikation sind, und die Resektion in unterschiedlichen Graden bei Patienten mit blutenden Ulci, die auf andere Therapien nicht ansprechen. Entsprechend der Erfahrung der Erfinder gibt es eine Wiederauftretensrate der Ulcuserkrankung nach der Vagotomie von 20 bis 30 % und ein ähnliches oder höheres Wiederauftreten des Ulcus nach der Resektion. Es ist bisher noch nicht bekannt, ob dieses Wiederauftreten in bezug zu setzen ist auf die pH-Werte oder ob pharmazeutische prophylaktische Maßnahmen dieses Wiederauftreten verringern können. Die Verwendung der vorliegenden Erfindung bei den post-operativen Nachuntersuchungen kann zur Lösung dieses Problems beitragen. Die pH-Wertüberwachung direkt nach der Operation kann die Wirksamkeit des operativen Vorganges selbst quantifizieren.

11. Quantitätsbestimmung der Medikation bei Ulcuserkrankung.

Die angenommenen und empfohlenen Dosierungen der H-2-Rezeptorblocker-Medikamente sind zur Behebung der Symptome und entsprechend der Heilungsgeschwindigkeiten von nachgewiesenen Ulcuserkrankungen oder Erosionen festgelegt. Es besteht die Möglichkeit, daß die im allgemeinen verwendeten Dosen höher sind als die für den einzelnen Patienten benötigten sind. Um eine optimale Therapie zu definieren, sollte ein quantitativer Endpunkt für das Ansprechen auf Medikamente verwendet werden und die vorliegende Erfindung versieht den Arzt mit einem derartigen Werkzeug. Auf diese Weise kann der Arzt eine minimale Dosierung des Medikaments festlegen, die eine optimale Reaktion (schnelle Heilung) bewirkt.

12. Behandlung von Oesophagitis.

Oesophagitis kann dem Patienten große Schmerzen und Unbehagen bereiten. Die Endoskopie kann das Vorhandensein oder Nichtvorhandensein von oesophagealen Varizen dokumentieren, aber es bleibt die Frage offen, ob sich die Oesophagitis auf den HCl Rückfluß vom Magen bezieht. Durch die Verabreichung von Antazida und/oder H-2-Rezeptorblockern kann festgestellt werden, ob eine Verringerung der Schmerzen auftritt, aber dies ist weder ein Beweis noch eine Bestimmung. Die pH-Sonde 1 der vorliegenden Erfindung kann in den Oesophagus eingeführt werden und die pH-Wertüberwachung kann die pH-Wertbeziehung zwischen den Symtomen und dem täglichen pH-Wert-Rhythmus und zwischen den Mahlzeiten und den Symptomen zeigen. Es können auch die optimalen Dosierungen aufgestellt werden und dem Arzt ein Hilfsmittel zur Bestimmung der Behandlung hinsichtlich der Wahl eines Antazidums oder eines H-2-Rezeptorblockers geben.

0200673

Patentansprüche

1. Tragbares Gerät zur Feststellung der Magenfunktion auf der Grundlage der Magensäure, g e k e n n z e i c h n e t   d u r c h eine pH-Sonde (1) zur kontinuierlichen Messung von pH-Werten im Oesophagus oder im Magen, einen Meßwertspeicher (9) zur Speicherung der einzelnen Meßwerte in Zuordnung der jeweiligen Uhrzeit, zu der sie gemessen werden, wobei eine elektronische Uhr (5) die Uhrzeit liefert, eine Anzeigeeinheit (7) zur Anzeige der Meßwerte, einen Drucker (8), der die Meßwerte während der Messung in Abhängigkeit von der Zeit ausgibt, ein Tastenfeld (6) zur Eingabe von Schwellenwerten für die pH-Werte und von Steuerbefehlen, einen Schwellenwertspeicher (10), in den die über das Tastenfeld (6) eingegebenen Schwellenwerte gespeichert sind, einen Vergleicher (11), der die Meßwerte mit dem jeweiligen Schwellenwert vergleicht, einen Signalgeber (12), der bei Über- bzw. Unterschreiten des jeweiligen Schwellenwertes für einen vorgebbaren Zeitraum ein optisches und/oder akustisches Signal abgibt und einer Steuereinheit (4) zur gesteuerten Weiterleitung der Meßwerte und Ausgangssignale.

2. Gerät nach Anspruch 1, dadurch gekennzeichnet, daß ein statistischer Rechner (17) vorgesehen ist, der abhängig von einem vorgebbaren Zeitraum den Mittelwert der gemessenen pH-Werte bildet.

0200673

3.          Geräte nach Anspruch 1 oder 2, dadurch gekennzeichnet, daß ein Ereignisspeicher (22) vorgesehen ist, der sowohl die vom Vergleicher (11) abgegebenen Signale als auch über das Tastenfeld eingebbare Ereignisse zeitrichtig speichert.

4. Gerät nach einem der Ansprüche 1 bis 3, dadurch gekennzeichnet, daß ein Anweisungsspeicher (21) vorgesehen ist, aus dem abhängig von der Zeit und/oder vom Vergleicher (11) abgegebenen Signalen Anweisungen zur Beeinflussung des pH-Wertes der Magensäure ausgelesen werden.

5. Gerät nach einem der Ansprüche 1 bis 4, dadurch gekennzeichnet, daß eine Medikamentenpumpe bzw. Infusionspumpe vorgesehen ist, die abhängig von der Zeit und/oder vom Ausgangssignal des Vergleichers durch die Steuereinheit (4) gesteuert wird.

6. Gerät nach einem der Ansprüche 1 bis 5, dadurch gekennzeichnet, daß dem Vergleicher (11) wahlweise das momentane Meßwertsignal oder ein vom statistischen Rechner (17) geliefertes den Mittelwert über eine bestimmte Zeit eingebendes Ausgangssignal zugeführt wird.

7. Verfahren zum Bestimmen des intraluminalen pH-Wertes eines Patienten mit dem Gerät nach Anspruch 1, gekennzeichnet durch folgende Schritte:
   a) Einführen der pH-Sonde in eine intraluminale Stelle des Patienten,

0200673

b) Messen der pH-Werte an dieser Stelle während eines vorbestimmten Zeitraums,

c) Speichern der pH-Werte und der Zeit, zu der sie gemessen werden,

d) Berechnen eines mittleren intraluminalen pH-Wertes für den vorbestimmten Zeitraum,

e) Eingeben eines vorgegebenen Schwellenwertes in den Schwellenwertspeicher,

f) Vergleichen mindestens eines gemessenen pH-Wertes oder mittleren pH-Wertes mit dem Schwellenwert,und

g) Anzeigen mindestens eines der gemessenen pH-Werte, der Zeit, an der er gemessen wurde, des mittleren pH-Wertes, des Zeitraums, über den der mittlere pH-Wert gemessen wurde,und des Schwellenwertes.

8. Verfahren zum Feststellen des Vorhandenseins einer Ulcus-Erkrankung unter Durchführung des Verfahrens nach Anspruch 7 während eines vorgewählten Zeitraums von 24 Stunden und mit folgenden zusätzlichen Schritten:

a) Bestimmen der Anzahl aller pH-Messungen während des 24 Stunden Zeitraumes,

b) Bestimmen der Anzahl der durchgeführten Messungen, deren Wert unter 1,5 liegt,

c) Vergleichen der Anzahl aller pH-Wertmessungen während des 24 Stunden Zeitraums mit der der unter 1,5 liegenden Meßwerte,

d) Bestimmen des gesamten Zeitraumes, bei dem die pH-Meßwerte während der 24 Stunden unter 1,5 liegen, und

e) Bestimmen der mittleren pH-Werte zwischen $15^{00}$ Uhr und $3^{00}$ Uhr.

9. Verfahren zum Bestimmen des täglichen Rhythmus der HCL-Produktion des Patienten unter Durchführung des Verfahrens nach Anspruch 7 für einen vorbestimmten Zeitraum von 24 Stunden und Berechnen von mittleren pH-Werten für vorbestimmte Zeitintervalle während der 24 Stunden.

10. Verfahren zum Bestimmen der Dosierung eines Medikaments zum Absenken des intraluminalen pH-Wertes und der Zeit, zu der der Patient das Medikament einnehmen soll, unter Durchführung des Verfahrens nach Anspruch 7 mit folgenden zusätzlichen Schritten:

   a) Messen des pH-Wertes vor dem Verabreichen des Medikaments,

   b) Verabreichen des Medikaments,

   c) Messen des pH-Wertes nach dem Einnehmen des Medikaments, und

   d) Festlegen der Dosierung des Medikaments unter Berücksichtigung der gemessenen pH-Werte und der Zeit, zu der das Medikament verabreicht wurde.

11. Verfahren zum Bestimmen, ob der Patient einen H-2 Rezeptorblocker oder ein Antacidum zur Steuerung des intraluminalen pH-Wertes nehmen soll unter Durchführung des Verfahrens nach Anspruch 8 mit folgenden zusätzlichen Schritten:

   a) Berechnen des mittleren pH-Wertes für den Zeitraum zwischen $6^{00}$ Uhr und $18^{00}$ Uhr (Tagesstunden),

   b) Berechnen des mittleren pH-Wertes für den Zeitraum zwischen $18^{00}$ Uhr und $6^{00}$ Uhr (Nachtstunden),

0200673

c) Berechnen der Pufferwirkung auf den pH-Wert nach Mahlzeiten,

d) Bestimmen, ob der Patient einen geringeren mittleren pH-Wert als normal während der Tagesstunden, einen mittleren pH-Wert größer als 1,5 während der Nachtstunden und eine geringe Pufferwirkung nach Mahlzeiten entfaltet, und

e) Bestimmen, ob der Patient während der Nachtstunden für längere Zeit einen pH-Wert von weniger als 1,5 und signifikante Pufferwirkungen nach Mahlzeiten entfaltet.

12. Verfahren zum Bestimmen der Wirkung von Mahlzeiten auf den täglichen pH-Wert-Rhythmus eines Patienten mit Ulcuserkrankung unter Verwendung des Verfahrens nach Anspruch 7 für einen vorbestimmten Zeitraum von mindestens 24 Stunden mit folgenden zusätzlichen Schritten:

a) Berechnen eines Mittel- und eines Median-pH-Wertes für einen Zeitraum von mindestens einer halben Stunden vor jeder Mahlzeit,

b) Berechnen des Mittel- und Median-pH-Wertes für einen Zeitraum von mindestens einer Stunde nach jeder Mahlzeit,

c) Vergleichen der für den Patienten bestimmten Mittel- und Medianwerte mit denen eines Gesunden während dergleichen Zeiträume.

13. Verfahren zum Bestimmen der Wirkung von Streß auf den intraluminalen pH-Wert eines Patienten unter Verwendung des Verfahrens nach Anspruch 7 für einen vorbestimmten Zeitraum von mindestens 24 Stunden mit folgenden zusätzlichen Schritten:

0200673

a) Berechnen eines mittleren und eines Median-pH-Wertes für einen Zeitraum, in dem der Patient unter Streß steht,

b) Berechnen eines mittleren und eines Median-pH-Wertes für einen Zeitraum, in dem der Patient nicht unter Streß steht,

c) Vergleichen der mittleren und Median-pH-Werte nach a) mit den mittleren und Median-pH-Werten nach b).

14. Verfahren zum Bestimmen, ob eine Oesophagitis sich auf die pH-Werte bezieht unter Verwendung des Verfahrens nach Anspruch 8, wobei die pH-Sonde für einen vorbestimmten Zeitraum von 24 Stunden im unteren Oesophagus positioniert wird.